# FASCICULE DE BREVET EUROPEEN

(11) **EP 2 822 446 B1**
(45) Date de publication et mention de la délivrance du brevet: **07.12.2016**
(21) Numéro de dépôt: 13715280.7
(22) Date de dépôt: 08.03.2013
(51) Int. Cl.: A61B 17/00, A61B 17/29, A61B 34/00, A61B 1/00, A61B 1/005, A61B 1/018

(54) **DISPOSITIF D'INSTRUMENTATION MOTORISÉ ET MODULABLE ET SYSTÈME D'ENDOSCOPE COMPRENANT UN TEL DISPOSITIF**
MOTORISIERTE UND MODULARE INSTRUMENTENVORRICHTUNG UND ENDOSKOPIESYSTEM MIT EINER SOLCHEN VORRICHTUNG
MOTORISED AND MODULAR INSTRUMENTATION DEVICE AND ENDOSCOPY SYSTEM COMPRISING SUCH A DEVICE

(30) Priorité: 08.03.2012 FR 1252109
(43) Date de publication de la demande: 14.01.2015
(73) Titulaire: Université de Strasbourg (Etablissement Public National à Caractère Scientifique, Culturel et Professionnel), 67000 Strasbourg (FR); Centre National de la Recherche Scientifique (CNRS) Etablissement Public à Caractère Scientifique et Technologique, 75016 Paris (FR)
(72) Inventeur: ZORN, Lucile, 68000 Colmar (FR); ZANNE, Philippe, 67480 Roppenheim (FR); NAGEOTTE, Florent, 68920 Wettolsheim (FR); DE MATHELIN, Michel, 67000 Strasbourg (FR)
(74) Mandataire: Nuss, Laurent
(86) Numéro de dépôt international: PCT/FR2013/050487
(87) Numéro de publication internationale: WO 2013/132194

(56) Documents cités:
- EP-A1- 1 987 789
- EP-A2- 1 980 213
- US-A1- 2005 119 522
- US-A1- 2008 046 122
- US-A1- 2008 103 358
- US-A1- 2010 274 078

## Description

La présente invention concerne le domaine des endoscopes, notamment flexibles, en particulier en relation avec la chirurgie endoluminale et transluminale, et a pour objets un dispositif d'instrumentation motorisé et modulable pour un endoscope ou un dispositif analogue à guide allongé rigide ou flexible et un système d'endoscope médical flexible comprenant au moins un tel dispositif.

De nombreuses réalisations d'endoscopes ou similaires, flexibles ou non, sont déjà connues dans l'état de l'art, par exemple par les documents DE 3928532, DE 19918961, EP 1 726 254 et DE 2504663. En outre le document EP 1 980 213 qui représente l'état de la technique le plus proche divulgue la préambule de la revendication 1.

De tels dispositifs comprennent généralement un corps allongé destiné à être introduit dans le corps du patient et comportant un ou plusieurs canaux fonctionnels pour le passage de fluides ou la mise en place d'instruments médicaux ou optiques, à structure allongée et généralement flexible, dont les extrémités pourvues d'outils, d'effecteurs ou analogues débouchent au niveau de l'extrémité distale du corps allongé de l'endoscope (situé dans le patient au niveau de la zone d'intervention ou d'intérêt).

L'extrémité proximale du corps allongé de l'endoscope, demeurant à l'extérieur du patient, est reliée mécaniquement et fonctionnellement à une poignée ou unité de commande qui permet de déplacer le corps allongé en translation et en rotation et d'infléchir ou de courber l'extrémité distale dudit corps.

En outre, ladite poignée est munie de passages pour l'introduction des instruments dans les canaux correspondants du corps allongé, chacun de ces instruments comportant ses propres moyens de commande (par exemple sous forme de poignées secondaires ou auxiliaires) permettant l'actionnement de l'outil, la flexion de l'extrémité distale de l'instrument et le déplacement en translation et/ou en rotation dudit instrument dans son canal, par l'opérateur.

Afin de limiter les efforts à fournir par ce dernier et rendre l'utilisation de l'endoscope plus aisée, plus sûre et plus simple, en particulier durant les interventions précises et de longue durée, il a été proposé de motoriser au moins certaines des manoeuvres, en particulier celles liées à la flexion des extrémités des instruments et à l'actionnement des outils.

De telles réalisations motorisées antérieures sont notamment connues par les documents DE 2504663, EP 0 078 017, DE 4213418, ainsi que par les publications suivantes :
"Design of a Telemanipulated System for Transluminal Surgery" (Conception d'un système télémanipulé pour la chirurgie transluminale), B. Bardou et al. : IEEE Engineering in Medicine and Biology Conference (EMBC 2009), Minneapolis, MN, Etats-Unis, septembre 2009 ;
"Control of a multiple sections flexible endoscopic system" (Contrôle d'un système endoscopique flexible à sections multiples), B. Bardou et al. : International Conference on Intelligent Robots and Systems (IROS), Taipei, Taïwan, pp. 2345-2350, octobre 2010 ;
"Design of a robotized flexible endoscope for natural orifice transluminal endoscopic surgery" (Conception d'un endoscope flexible robotisé pour la chirurgie transluminale par orifice naturel), B. Bardou et al., : Computational Surgery and Dual Training", M. Garbey et al. (Eds.), Chapitre 9, pp. 155-170, Springer, ISBN : 978-1-4419-1122-3, 2010.

Or, il existe actuellement une demande pour motoriser également les déplacements des instruments, voir le déplacement de l'endoscope lui-même, en particulier pour permettre une commande à distance, sans action physique directe de l'opérateur, des instruments et, le cas échéant, de l'endoscope, après leur mise en place initiale.

En outre, il serait avantageux que les instruments présentent également une constitution compacte au niveau de leurs parties demeurant à l'extérieur, intègrent des parties mobiles isolées de l'environnement extérieur, et puissent être interchangés aisément et que le même système endoscopique puisse accueillir des instruments de différents types, avec possibilité d'actionnement et de déplacement motorisés.

La présente invention a pour but de fournir une solution répondant au moins aux principaux besoins exposés précédemment.

A cet effet, l'invention a pour objet un dispositif d'instrumentation motorisé et modulable pour un endoscope ou analogue, en particulier un endoscope flexible, comprenant au moins une unité opérationnelle montée sur au moins une structure support, préférentiellement articulée, la ou chaque unité intégrant un module d'instrument médical motorisé comprenant, d'une part, un instrument médical allongé avec un effecteur ou outil disposé à une extrémité distale, la portion d'extrémité correspondante de l'instrument étant apte à subir une flexion ou un cintrage dans au moins un plan et/ou selon au moins une direction et, d'autre part, au moins un moyen d'actionnement motorisé commandant le fonctionnement de l'outil ou de l'effecteur et/ou la flexion ou le cintrage de la portion d'extrémité distale, ce par l'intermédiaire de moyens de transmission s'étendant dans le corps allongé de l'instrument médical, ce dernier étant en outre apte à être soumis à des déplacements en translation dans la direction de son axe longitudinal et à des déplacements en rotation autour de cet axe.

Ce dispositif d'instrumentation est caractérisé en ce que le ou les moyen(s) d'actionnement est(sont) monté(s) dans un corps creux relié à l'extrémité proximale de l'instrument médical et en ce que ledit corps creux est lui-même disposé, avec guidage en rotation autour d'un axe médian propre prolongeant l'axe longitudinal de l'instrument médical, dans un boîtier de réception, ce dernier étant monté avec faculté de déplacement, au moins en translation selon la direction de l'axe médian du corps creux, sur la structure support, les déplacements du corps creux et du boîtier de réception étant motorisés.

L'invention a également pour objet un système endoscopique flexible comprenant, d'une part, un corps allongé avec au moins un, préférentiellement au moins deux, canal(aux) fonctionnel(s) longitudinal(aux) destiné(s) à recevoir chacun un instrument médical dont l'extrémité distale portant l'effecteur ou l'outil peut déboucher de l'extrémité distale dudit corps allongé et, d'autre part, une unité ou poignée de commande reliée à l'extrémité proximale du corps allongé et comportant, pour le ou chaque instrument médical, une ouverture d'introduction se prolongeant par un canal longitudinal correspondant, ladite unité ou poignée intégrant des moyens d'actionnement contrôlant la flexion ou le cintrage de la portion d'extrémité distale au moins du corps allongé.

Ce système endoscopique est caractérisé en ce qu'il comprend en outre au moins un dispositif d'instrumentation motorisé et modulable tel qu'évoqué ci-dessus, dont l'instrument médical est disposé dans le ou un canal fonctionnel avec faculté de déplacement en translation et en rotation.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :
Les figures 1A et 1B sont des vues en perspective, selon deux directions sensiblement opposées, d'un système d'endoscope flexible selon un premier mode de réalisation de l'invention comprenant un dispositif d'instrumentation selon l'invention et deux structures support séparées ;
La figure 2 est une vue en perspective d'une unité opérationnelle faisant partie du dispositif représenté sur les figures 1A et 1B;
La figure 3 est une vue en perspective à une autre échelle et selon une autre direction de l'unité opérationnelle représentée figure 2, le boîtier de réception étant ouvert ;
La figure 4 est une vue en perspective du boîtier de réception représenté figure 3, le module d'instrument médical étant enlevé ;
La figure 5 est une vue en perspective du module d'instrument représenté figure 3, avec ses moyens de guidage et d'entraînement en rotation ;
La figure 6 est une représentation d'un module d'instrument similaire à celle de la figure 5, les moyens de guidage et d'entraînement n'étant pas représentés ;
La figure 7 est une vue en perspective du module d'instrument représenté figures 5 et 6, illustrant les moyens d'actionnement contrôlant la flexion de la portion d'extrémité de l'instrument médical, une partie du corps creux étant enlevée ;
La figure 8 est une vue en perspective similaire à celle de la figure 7, illustrant les moyens d'actionnement commandant l'outil ;
Les figures 9A et 9B sont des vues partielles illustrant la portion d'extrémité d'un outil faisant partie d'un instrument médical selon l'invention, illustrant les possibilités de flexion dans un plan ;
Les figures 10A et 10B sont des schémas cinématiques de principe d'un système d'endoscope tel que représenté figures 1A et 1B, selon deux variantes d'un premier mode de réalisation de l'invention ;
Les figures 11A et 11B sont des schémas cinématiques de principe de deux variantes d'un autre mode de réalisation d'un système d'endoscope tel que représenté figures 1A et 1B ;
La figure 12 est une vue en perspective, similaire à celle de la figure 1B, représentant partiellement un système d'endoscope flexible (les modules d'instruments ne sont pas représentés), en accord avec une autre variante de réalisation du montage de la poignée de commande, et,
La figure 13 est une vue en perspective, similaire à celle de la figure 1B, d'un système d'endoscope flexible selon un autre mode de réalisation de l'invention, comprenant un dispositif d'instrumentation selon l'invention et correspondant aux schémas cinématiques des figures 11A et 11B, avec une structure support unique.

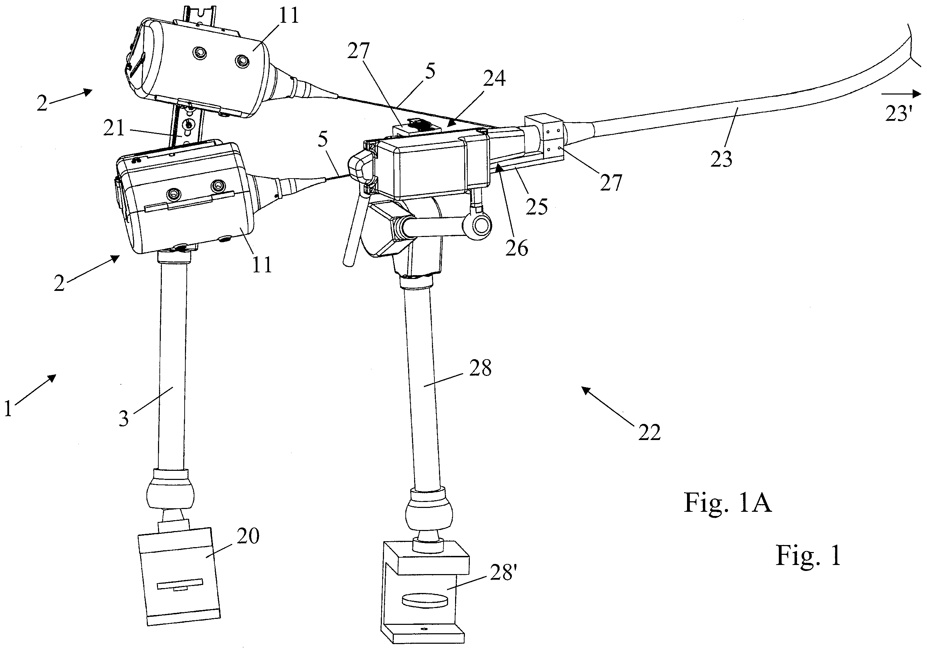

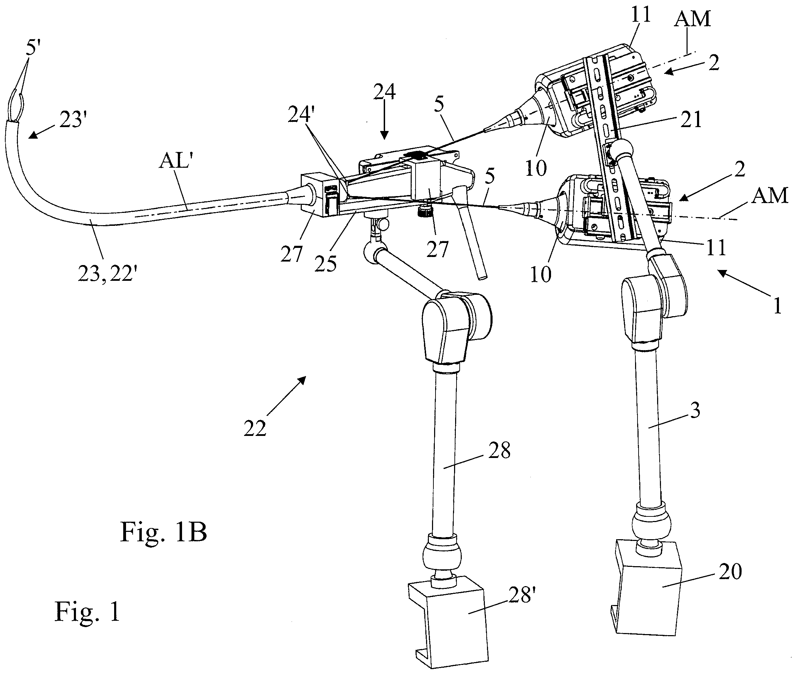

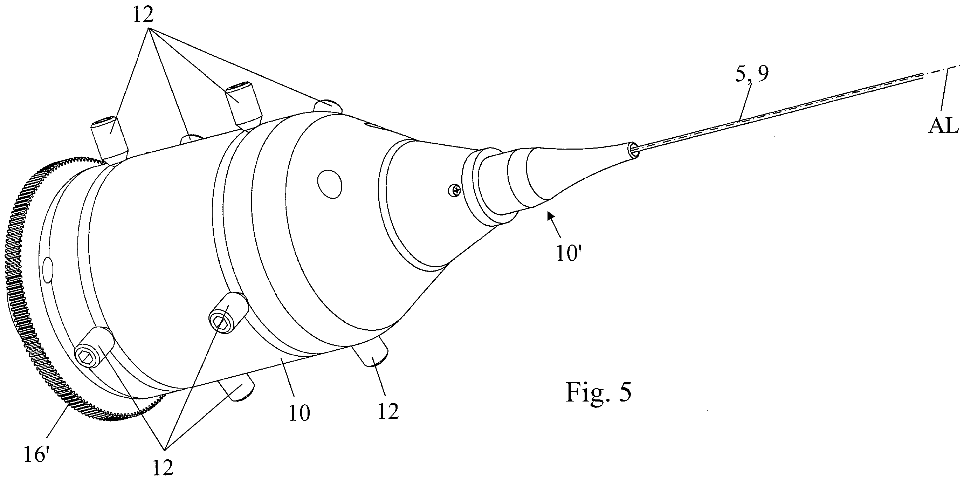

Les figures 1 à 8 illustrent, pour certaines seulement partiellement, un dispositif 1 d'instrumentation motorisé et modulable pour un endoscope ou analogue, en particulier un endoscope flexible, avec ou sans moyen de vision.

Ce dispositif 1 comprend au moins une unité opérationnelle 2 montée sur au moins une structure support 3, préférentiellement articulée. La ou chaque unité 2 intègre un module d'instrument médical motorisé 4 comprenant, d'une part, un instrument médical 5 allongé avec un effecteur ou outil 6 disposé à une extrémité distale, la portion d'extrémité 5' correspondante de l'instrument 5 étant apte à subir une flexion ou un cintrage dans au moins un plan et/ou selon au moins une direction et, d'autre part, au moins un moyen d'actionnement motorisé 7, 7' commandant le fonctionnement de l'outil ou de l'effecteur 6 et/ou la flexion ou le cintrage de la portion d'extrémité 5' distale, ce par l'intermédiaire de moyens de transmission 8, 8' s'étendant dans le corps allongé 9 de l'instrument médical, ce dernier étant en outre apte à être soumis à des déplacements en translation dans la direction de son axe longitudinal AL et à des déplacements en rotation autour de cet axe.

Conformément à l'invention, le ou les moyen(s) d'actionnement 7, 7' est(sont) monté(s) dans un corps creux 10 relié à l'extrémité proximale 5" de l'instrument médical 5 et ledit corps creux 10 est lui-même disposé, avec guidage en rotation autour d'un axe médian propre AM prolongeant l'axe longitudinal AL de l'instrument médical 5, dans un boîtier de réception 11, ce dernier étant monté avec faculté de déplacement, au moins en translation selon la direction de l'axe médian AM du corps creux 10, sur la structure support 3, les déplacements du corps creux 10 et du boîtier de réception 11 étant motorisés.

Ainsi, les moyens d'actionnement 7, 7' sont regroupés dans une enceinte fermée et séparés du milieu extérieur et le corps creux 10 forme avec l'instrument médical 5 qui lui est raccordé un module 4 structurel et fonctionnel, à constitution unitaire. De plus, le ou chaque module d'instrument médical 4 peut, dans sa totalité, être déplacé en rotation et en translation par rapport à la structure support 3 qui le porte, présentant ainsi au moins les deux degrés de liberté dont font état les instruments médicaux actuels pourvus de poignées de manipulation propres. Toutefois, grâce à l'invention, les déplacements possibles selon ces degrés de liberté sont eux aussi motorisés.

De ce fait, l'invention propose notamment des moyens techniques spécifiques, adaptés au domaine d'application visé, permettant de fournir une manoeuvrabilité améliorée, une assistance efficace à l'opérateur et une manipulation plus rapide du dispositif d'instrumentation, et donc du système endoscopique qu'il équipe.

Dans la présente, on entend par "motorisés" des moyens d'actionnement 7, 7' dont la force motrice ou l'entraînement initial(e) n'est pas d'origine humaine ou manuelle, mais résulte d'une transformation d'énergie en mouvement, éventuellement sous contrôle d'un opérateur.

Ainsi, les moyens d'actionnement 7, 7' comprennent ou correspondent à des actionneurs tels que par exemple des moteurs ou des vérins électriques, pneumatiques ou hydrauliques (à eau).

La description qui suit présente plus particulièrement des moyens d'actionnement 7, 7' de type électrique, mais des moyens d'actionnement utilisant une autre énergie peuvent également être envisagés sans sortir du cadre de l'invention.

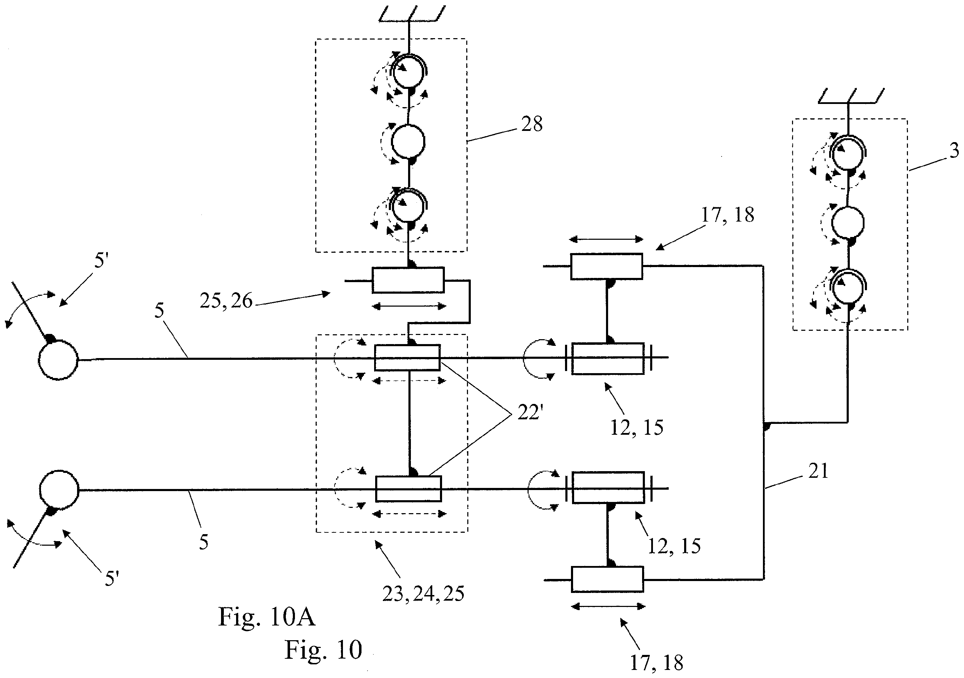

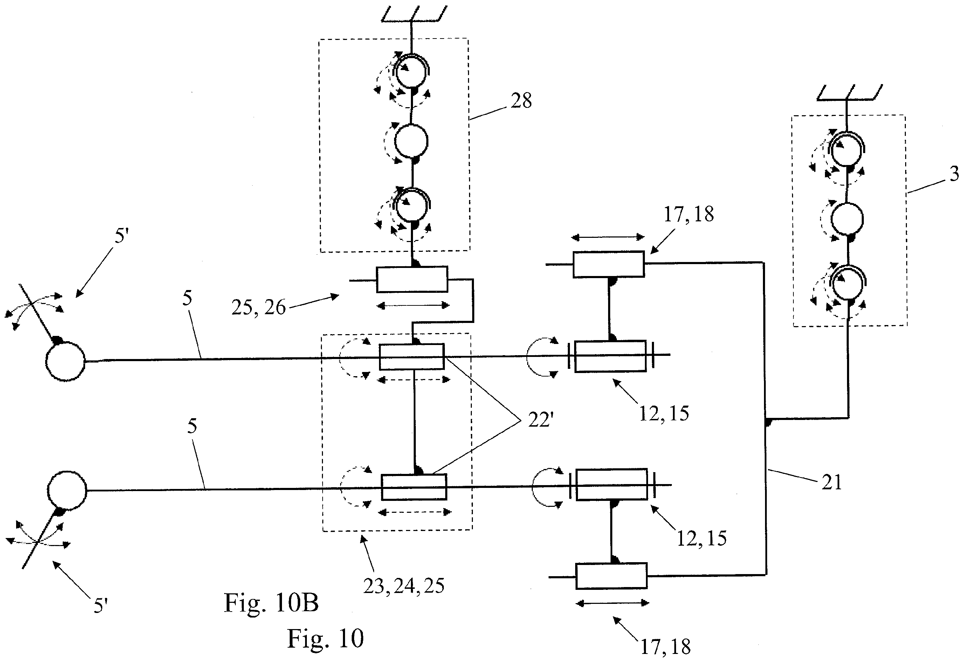

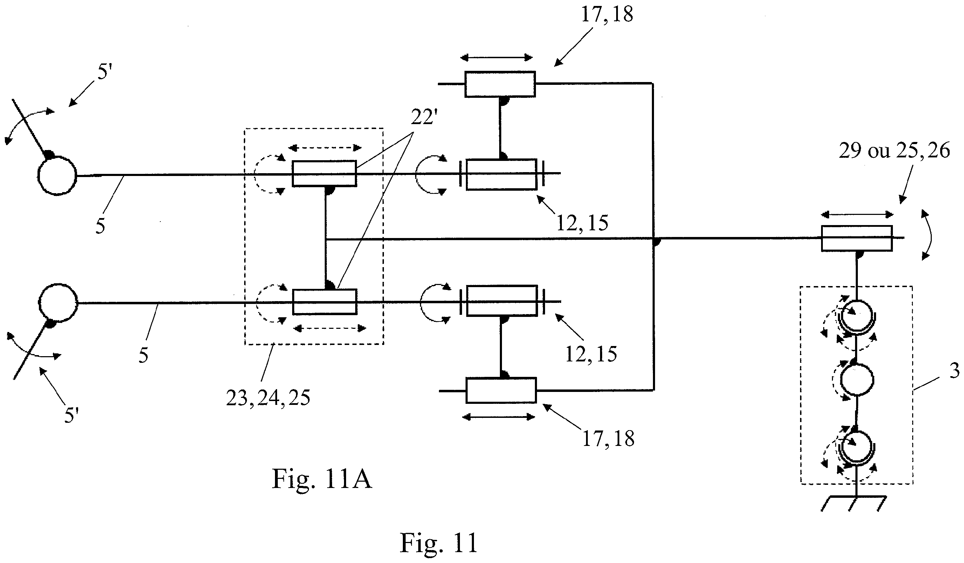

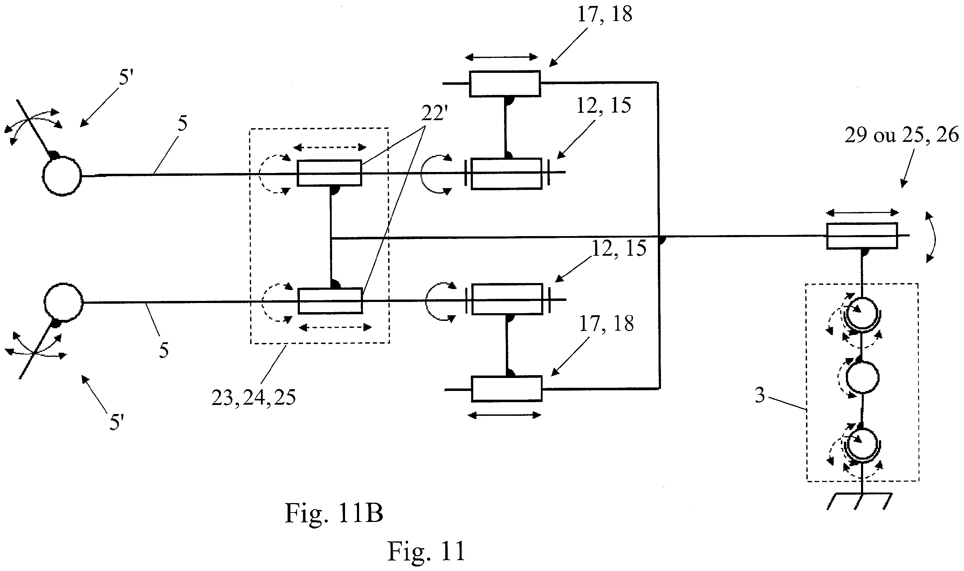

De manière générale sur les figures 10 et 11 des dessins annexés, les flèches symbolisant les mouvements de translation et de rotation sont représentées en traits continus pour les mouvements/liaisons/articulations motorisés et en traits pointillés pour les liaisons/articulations passives.

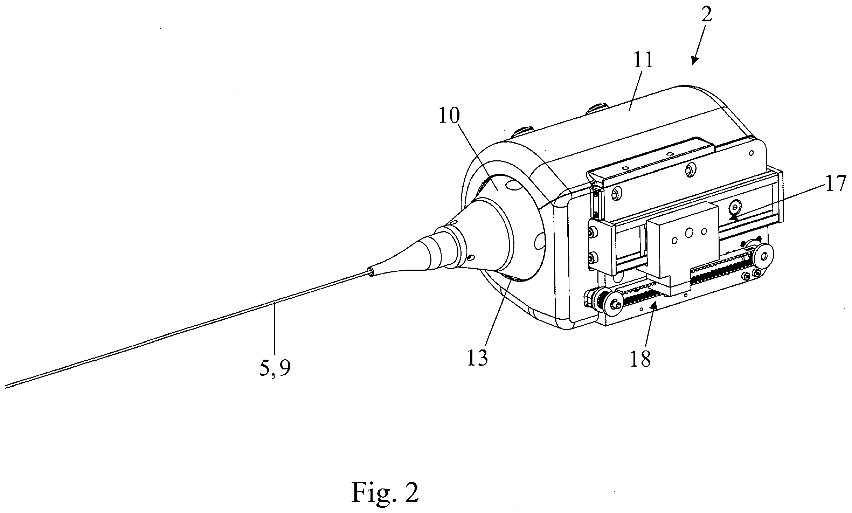

En accord avec un mode de réalisation avantageux de l'invention, ressortant plus particulièrement des figures 2 et 5 à 9, le corps creux 10, qui délimite avantageusement une enceinte sensiblement étanche, présente, au moins extérieurement, une structure de révolution autour de son axe médian AM, avantageusement cylindrique circulaire et préférentiellement en forme d'obus avec une extrémité effilée 10' constituant une interface de raccordement avec le corps 9 de l'instrument médical 5. D'autre part, le boîtier de réception 11 comporte un arrangement d'au moins deux paliers à roulement 12 espacés, à structure continue ou discontinue, assurant le guidage en rotation du corps creux 10 dans le boîtier 11.

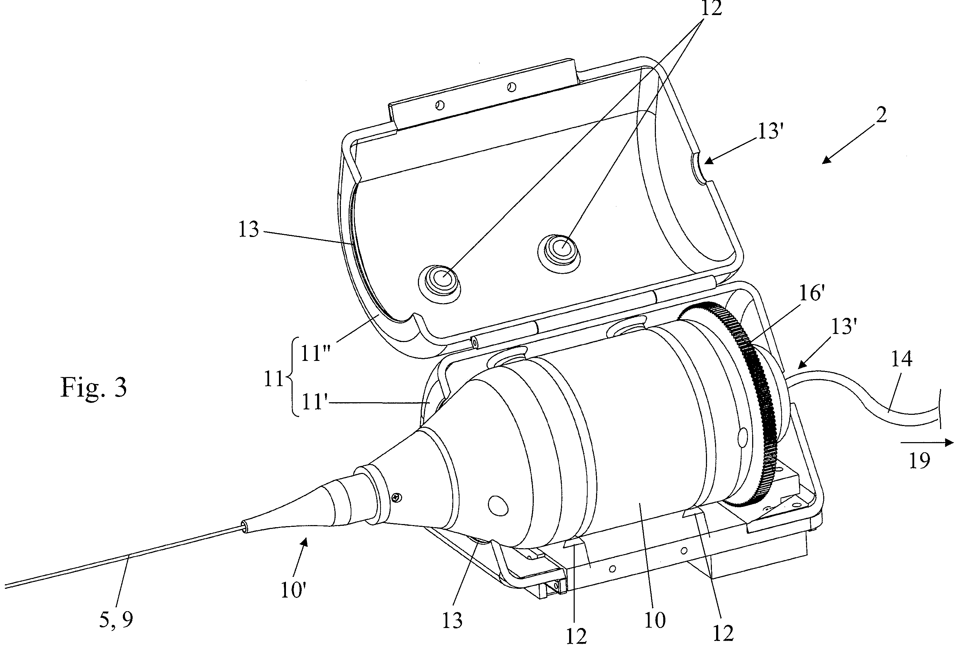

Comme le montrent les figures 3 à 5, les paliers 12 peuvent consister en des vis à bille intégrées au boîtier 11.

Bien entendu, le corps creux 10 est préférentiellement identique en termes de conformation extérieure pour les différents types d'instruments 5 de manière à pouvoir mettre en oeuvre les mêmes boîtiers de réception 11, bien que les moyens d'actionnement 7, 7' que chaque corps 10 renferme puissent être différents.

Le raccordement entre les corps 10 et 9 est suffisamment rigide pour autoriser une transmission des forces de traction/poussée et des couples entre eux durant leur manipulation, préférentiellement sans rupture d'étanchéité entre eux, et permet d'aboutir à un module d'instrument 4 unitaire.

L'étanchéité du corps creux 10 (par exemple formé de deux demi-coquilles, en un matériau adapté à un usage médical, assemblées de manière étanche) permet d'isoler de manière sûre les moyens d'actionnement 7, 7', ainsi que les parties des moyens de transmission 8, 8' (par exemple du type câbles, tiges ou analogues) externes au corps 9 de l'instrument 5, du milieu extérieur et de former avec ledit corps 9 un ensemble clos.

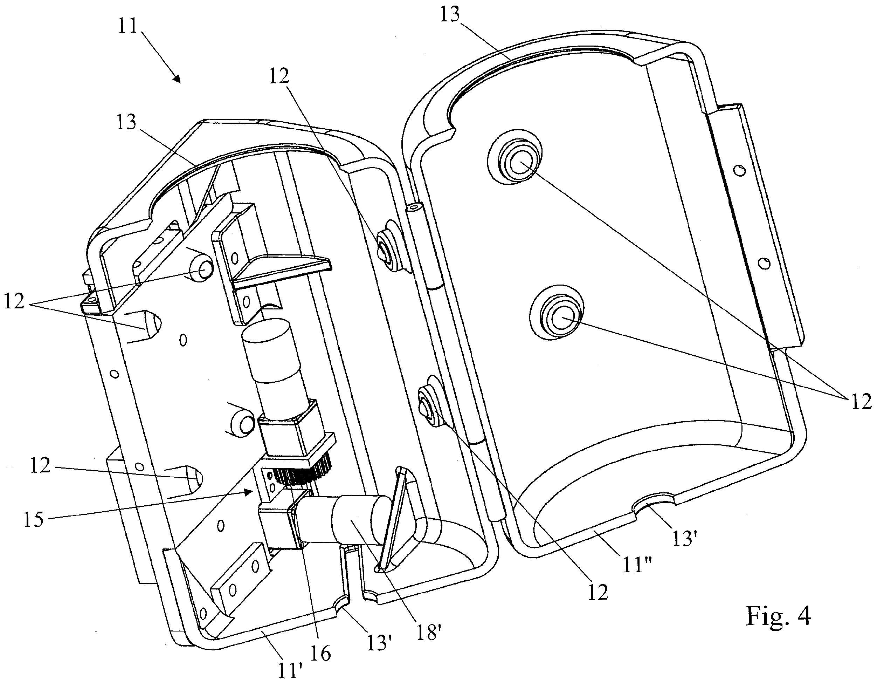

Comme le montrent notamment les figures 3 et 4, le boîtier de réception 11 peut consister en une coque en au moins deux parties 11', 11 ", par exemple deux parties mutuellement articulées dont l'une 11" forme un couvercle, autorisant l'introduction et l'extraction d'un corps creux 10 en position ouverte, ladite coque comportant deux ouvertures opposées 13, 13' destinées respectivement, d'une part, au passage de l'instrument médical 5 ou d'une partie d'extrémité 10' du corps creux 10, et, d'autre part, au passage des lignes 14 d'alimentation et/ou de commande du ou des moyen(s) d'actionnement 7, 7' commandant l'outil 6 et/ou l'extrémité distale 5' flexible.

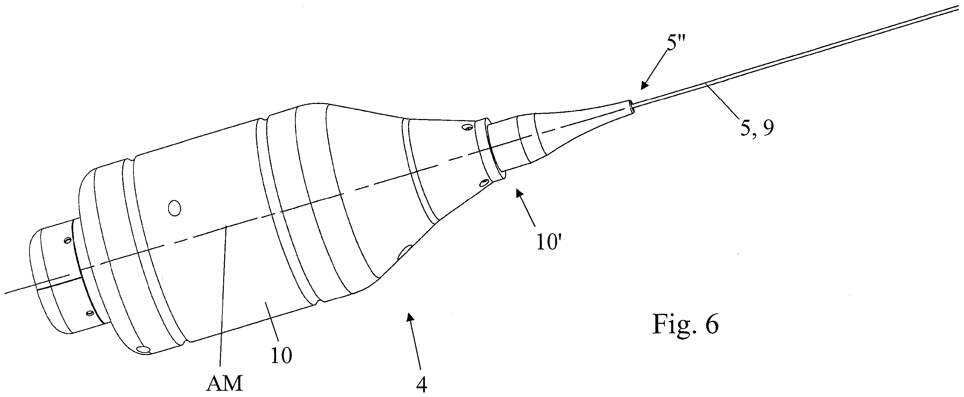

Les lignes 14 peuvent, par exemple, traverser l'enceinte du corps creux 10 au niveau d'un raccord ou d'une interface de passage rotative étanche 10" (figures 6 à 8).

Selon une caractéristique de l'invention, illustrée aux figures 3 à 5 des dessins annexés, le boîtier de réception 11 comporte un mécanisme motorisé 15 d'entraînement contrôlé en rotation du corps creux 10 autour de son axe médian AM, par exemple formé d'un pignon denté motorisé 16 engrenant avec une couronne dentée 16' s'étendant périphériquement autour dudit corps creux 10, la liaison entre ce dernier et l'instrument médical 5 étant apte à transmettre cette rotation au corps allongé 9 dudit instrument 5.

Ainsi, l'ensemble des composantes fonctionnelles participant à la rotation contrôlée du corps creux 10, et donc de l'instrument 5, est logé à l'intérieur du boîtier 11, lequel peut éventuellement également former une enceinte étanche en prévoyant des joints au niveau des ouvertures 13 et 13', compatible avec la rotation du corps creux 10 (joints à lèvres toriques ou analogues).

En outre, en prévoyant une couronne dentée 16' sur le pourtour extérieur du corps creux 10, il est possible de transmettre à ce dernier un couple important pour une motorisation de puissance donnée du pignon 16, compte tenu du rapport de diamètre important entre ledit pignon et ladite roue.

Pour la motorisation de la translation de l'instrument 5, il peut être prévu que le ou chaque boîtier de réception 11 soit monté sur la ou une structure support 3 par l'intermédiaire d'une liaison coulissante 17, par exemple une liaison à glissière, à coulisseau ou à chariot, sensiblement selon la direction de l'axe longitudinal AL de l'instrument médical allongé 5 qu'il renferme, le déplacement du boîtier de réception 11 par rapport à la structure support 3 concernée (selon AL) étant contrôlé par un mécanisme d'entraînement motorisé 18, préférentiellement intégré audit boîtier 11 (figures 1B et 2), par exemple un mécanisme à crémaillère ou à courroie crantée dont le pignon motorisé est entraîné par un actionneur électrique 18' logé dans le boîtier 11 (Fig. 4).

Bien entendu, les composantes extérieures de la liaison coulissante 17 et du mécanisme d'entraînement peuvent être recouvertes par un capot de protection (non représenté).

En transférant le déplacement en translation de l'instrument 5 au niveau du boîtier de réception 11, avantageusement avec une motorisation intégrée à l'intérieur de ce dernier, il est possible de simplifier la réalisation des deux motorisations (découplage entre les déplacements en translation et en rotation), de réduire l'encombrement du corps creux 10 et de simplifier la réalisation des mécanismes qu'il intègre.

Comme le montrent les figures 1A et 1B des dessins annexés, la ou chaque structure support 3 peut consister en un bras articulé dont une extrémité est pourvue d'un organe 20 de fixation ou d'assemblage rigide et amovible avec un équipement d'une salle d'opération, par exemple une table d'opération, et dont l'autre extrémité porte au moins un, préférentiellement deux, boîtier(s) de réception 11, par exemple par l'intermédiaire d'une entretoise support ou d'une pièce analogue 21 autorisant un montage orienté et éventuellement mutuellement espacé, lorsque plusieurs boîtiers de réception 11 sont présents.

Fonctionnellement, le bras 3 présente un nombre important de degrés de liberté, préférentiellement au moins six, autorisant un prépositionnement (position et orientation) de l'unité 2 ou de chaque unité 2 par rapport à la table d'opération (non représentée) et par rapport à l'endoscope 22.

Physiquement, le bras 3 peut présenter un nombre variable d'articulations, éventuellement de différents types (pivot, rotule), associées éventuellement à des portions télescopiques, lesdites articulations pouvant être bloquées chacune rigidement (de manière amovible) dans une position donnée.

En outre, comme déjà indiqué, il peut comprendre également un organe 20 de fixation amovible avec un autre élément d'équipement du site d'installation (par exemple salle d'opération).

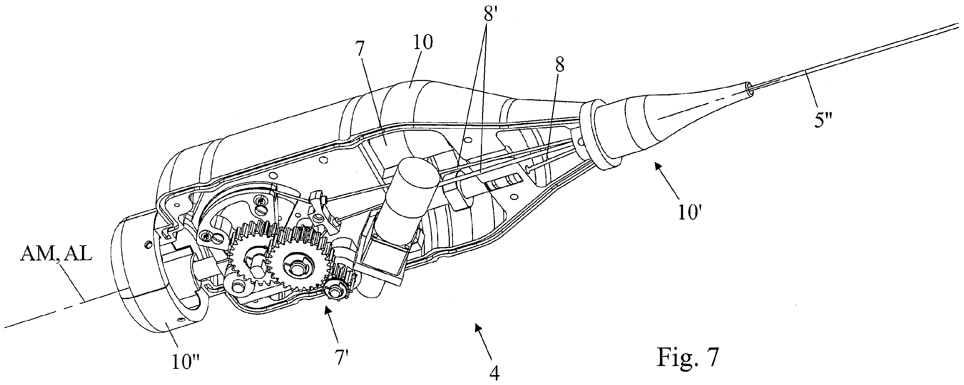

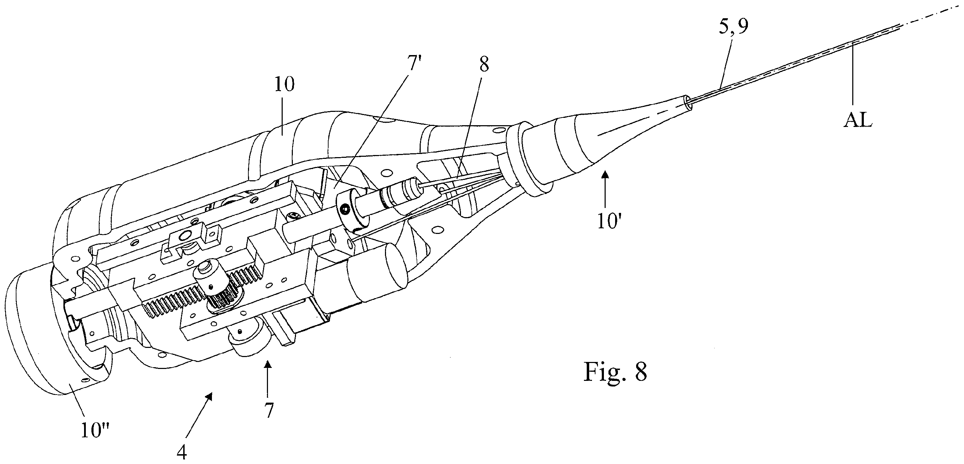

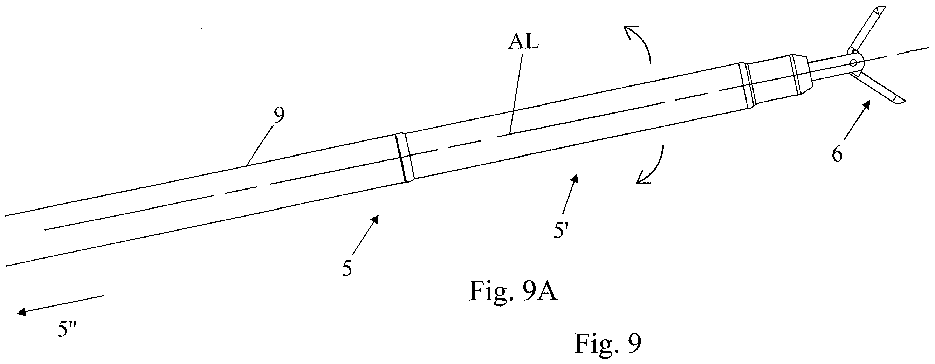

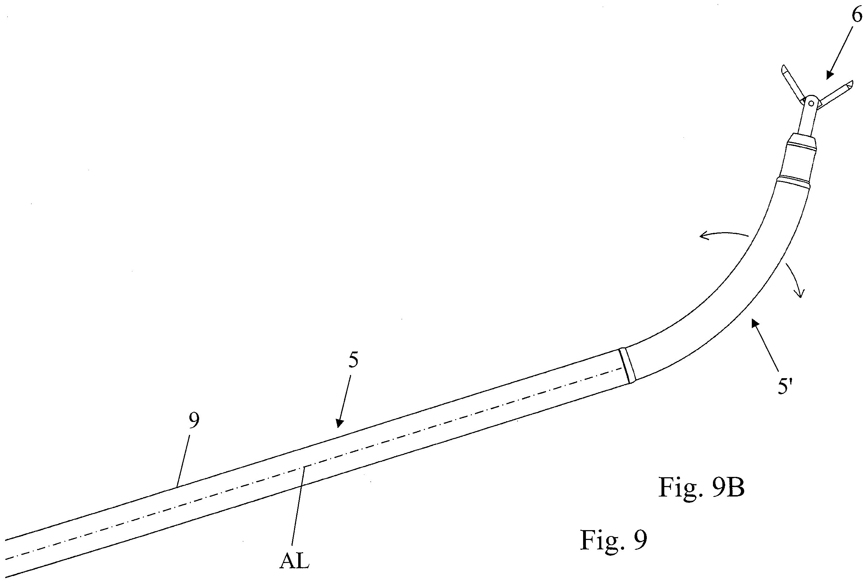

Les figures 7 et 8 illustrent, à titre d'exemple, un mode pratique de réalisation des moyens 7, 7', 8, 8' permettant la commande de l'outil 6 et la flexion de la portion d'extrémité distale 5' de l'instrument 5 dans un plan (en relation avec les figures 9A et 9B et les schémas cinématiques des figures 10A et 11A).

Ainsi, le moyen d'actionnement 7 (figure 8) assurant l'actionnement de l'outil 6 (ici une pince) peut consister en un moteur rotatif transmettant son mouvement de sortie, par l'intermédiaire d'un engrenage hélicoïdal par exemple, à un mécanisme à crémaillère dont la partie mobile en translation est solidaire du câble de commande 8 relié à la pince 6.

Le moyen d'actionnement 7' (figure 7) assurant la flexion de la portion d'extrémité 5' peut, quant à lui, comprendre un moteur rotatif transmettant son mouvement de sortie, par l'intermédiaire d'un engrenage hélicoïdal et éventuellement d'un train d'engrenages réducteur par exemple, à une pièce d'entraînement bidirectionnel des câbles de commande antagonistes 8' aptes à provoquer la flexion de l'extrémité 5'.

De manière alternative, il peut être prévu, comme cela ressort schématiquement des variantes représentées aux figures 10B et 11B, que le corps creux 10 intègre des moyens d'actionnement 7' et des moyens de transmission 8' permettant de réaliser une flexion ou un cintrage de l'extrémité distale 5' de l'instrument médical 5 dans deux plans sécants, préférentiellement perpendiculaires entre eux. L'homme du métier comprend qu'il suffit pour cela de doubler les moyens 7' et 8' présents dans le corps creux 10.

Avantageusement, le dispositif 1 comprend également, pour chaque module d'instrument 4 et éventuellement pour chaque boîtier de réception 11, une unité de commande 19 dédiée (non spécifiquement représentée) pour le contrôle des moyens d'actionnement 7, 7' et, le cas échéant, du mécanisme d'entraînement motorisé 18 et/ou du mécanisme 15 de rotation motorisée, la ou chaque unité de commande étant éventuellement asservie et/ou apte à traiter des signaux de mesure fournis par un ou des capteur(s) ou détecteur(s), par l'intermédiaire des lignes 14.

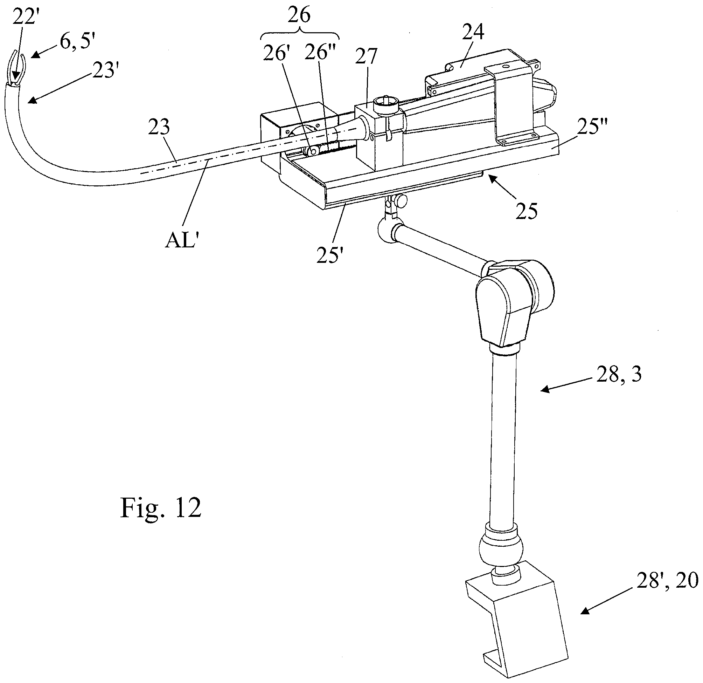

L'invention concerne également, comme le montrent à titre d'exemples les figures 1A, 1B, 10A, 10B, 11A et 11B, et partiellement la figure 12, un système endoscopique flexible 22.

Ce système comprend, d'une part, un corps allongé 23 avec au moins un, préférentiellement au moins deux, canal(aux) fonctionnel(s) longitudinal(aux) 22' destiné(s) à recevoir chacun un instrument médical 5 dont l'extrémité distale 5' portant l'effecteur ou l'outil 6 peut déboucher de l'extrémité distale 23' dudit corps allongé 23 et, d'autre part, une unité ou poignée de commande 24 reliée à l'extrémité proximale du corps allongé 23 et comportant, pour le ou chaque instrument médical 5, une ouverture d'introduction 24' se prolongeant par un canal longitudinal correspondant.

Cette unité ou poignée 24 intègre des moyens d'actionnement contrôlant la flexion ou le cintrage de la portion d'extrémité distale 23' au moins du corps allongé 23.

Ce système endoscopique 22 est caractérisé en ce qu'il comprend en outre au moins un dispositif 1 d'instrumentation motorisé et modulable tel que décrit ci-dessus, dont l'instrument médical 5 est disposé dans le ou un canal fonctionnel 22' avec faculté de déplacement en translation et en rotation.

De manière avantageuse, mais non limitativement, et comme le montrent les figures des dessins annexés, le ou chaque dispositif d'instrumentation 1 comprend au moins deux unités opérationnelles 2, dont les boîtiers de réception 11 sont montés sur une entretoise 21 ou analogue par l'intermédiaire d'une liaison coulissante 17, préférentiellement motorisée, la disposition relative des unités opérationnelles 2 par rapport à la poignée 24 ou analogue, maintenue par un moyen de fixation 27 fixe ou solidaire d'une platine 25 avec au moins un degré de liberté, étant réglée de telle manière que les axes médians AM des corps creux 10 sont sensiblement alignés avec les ouvertures 24' d'introduction des instruments médicaux 5 de la poignée 24 ou analogue.

En accord avec une possible caractéristique avantageuse du système endoscopique selon l'invention, et comme le montrent les figures 1A, 1B et 12, la poignée de commande 24 peut être fixée sur une platine de montage 25, dont le positionnement spatial est réglable grâce à une structure support 28, 3 articulée.

Selon une première variante de réalisation, ressortant des figures 1A et 1B, la poignée 24 est fixée rigidement à la platine de montage 25 par des moyens de fixation 27 amovibles tels que par exemple une bride de fixation annulaire associée à une aile ou un doigt de rétention par serrage.

Afin de fournir des possibilités supplémentaires de déplacement pour le système endoscopique, il peut être prévu que ladite poignée 24 puisse être déplacée en translation, sensiblement selon la direction de l'axe longitudinal AL' du corps allongé flexible 23, de manière contrôlée, préférentiellement motorisée.

Plus précisément, et comme le montre la figure 12, la platine de montage 25 peut comprendre une embase 25' solidarisée à la structure support 28, 3 et un chariot 25" comportant des moyens 27 de fixation rigide et amovible pour la poignée 24, le chariot 25" étant guidé en translation par rapport à l'embase 25' et déplacé par rapport à ce dernier grâce à un moyen 26 d'entraînement motorisé, par exemple sous la forme d'un pignon motorisé 26' engrenant soit avec un rail denté ou une crémaillère solidaire du ou formé sur le chariot 25", soit avec une courroie crantée 26" déplaçant ledit chariot 25".

Les moyens de fixation 27 mis en oeuvre sur la variante de la figure 12 peuvent être similaires à ceux des figures 1A et 1B.

Bien entendu, et bien que non représenté, il peut également être envisagé, de manière similaire, de fournir une possibilité de déplacement en rotation par rapport à l'axe AL' de la poignée 24, éventuellement aussi de type motorisé.

Préférentiellement, les déplacements du ou des module(s) d'instrument médical 4 et ceux de la poignée de commande 24 sont mutuellement asservis.

Cet asservissement peut être de nature mécanique (montage sur un même support) ou être de nature électronique en prévoyant un système de pilotage commandant simultanément le déplacement des modules 4 et de la poignée 24.

Selon une première variante de réalisation ressortant des figures 1A, 1B, 10A et 10B, la platine 25 est portée par une structure support 28 distincte et indépendante de la ou des structure(s) support(s) 3 portant le ou les dispositif(s) d'instrumentation 1, par exemple par un bras articulé dont une extrémité est pourvue d'un organe 28' pour la fixation ou l'assemblage rigide et amovible avec un équipement d'une salle d'opération, par exemple une table d'opération.

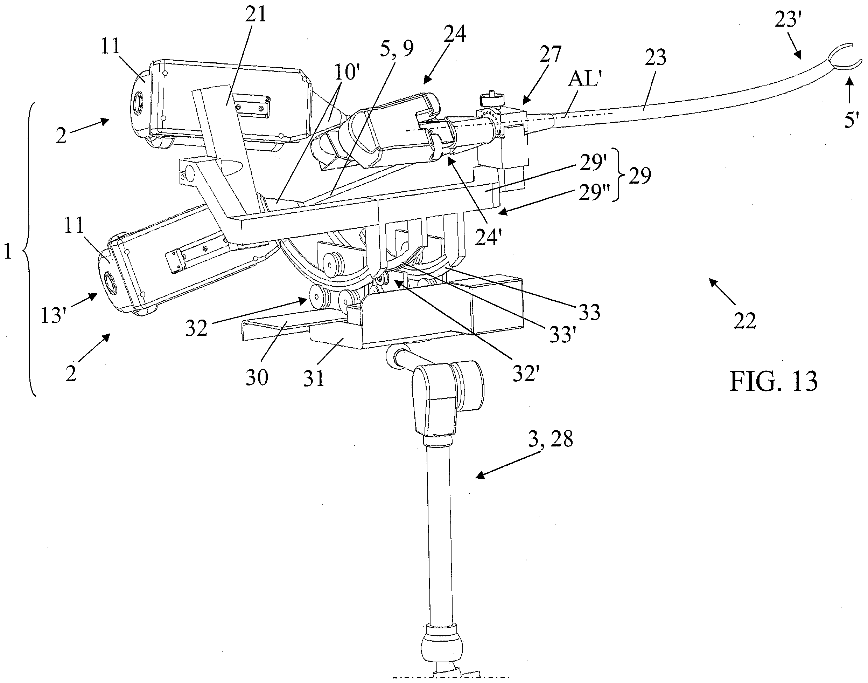

Selon une seconde variante de réalisation ressortant schématiquement des figures 11A et 11B, et illustrée sous forme d'exemple de mise en oeuvre pratique sur la figure 13, la platine 25 ou ledit au moins un moyen de fixation 27 est porté(e) par la même structure support 3 que celle portant le ou les boîtiers de réception 11 du dispositif d'instrumentation 1.

Plus précisément, et comme l'illustre à titre d'exemple pratique la figure 13, il peut être prévu que ledit au moins un dispositif 1 d'instrumentation motorisé et modulable et ladite unité ou poignée de commande 24 sont portés par une unique structure support 3, par exemple sous la forme d'un bras ou d'un pied articulé, préférentiellement à articulations multiples. De plus, ledit dispositif 1 et ladite poignée 24 sont montés sur ladite structure support 3 par l'intermédiaire d'un châssis porteur 29 fournissant une configuration de positionnement mutuel adaptée pour les unités opérationnelles 2 et la poignée 24 et pouvant être déplacé, de manière contrôlée, en rotation et en translation par rapport à la structure support 3, préférentiellement sensiblement autour et selon l'axe longitudinal AL' du corps allongé 23 du système endoscopique 22.

On assure ainsi deux degrés de liberté motorisés à l'ensemble du système endoscopique 22. L'homme du métier constate que cette variante de réalisation peut être dérivée de celle des figures 1A et 1B, en assimilant le châssis porteur 29 à une platine 25 qui supporterait également les unités opérationnelles 2.

Les déplacements en rotation et en translation sont avantageusement réalisés de manière indépendante et séparée.

Ainsi, les moyens de déplacement du châssis porteur 29 par rapport à la structure support 3 comprennent, d'une part, un chariot ou un patin 30 mobile en translation, de manière motorisée, par rapport à une platine 31 solidaire de la structure support 3, et, d'autre part, des ensembles 32, 32' de guidage et d'entraînement en rotation du châssis porteur 29 fixés sur ce chariot ou patin 30.

Selon une variante pratique d'exécution de l'invention, le châssis support 29 comprend, d'une part, une superstructure 29' sur laquelle sont installés, éventuellement par l'intermédiaire d'une liaison articulée pouvant être bloquée en position, une entretoise support 21 ou analogue pour les unités opérationnelles et le moyen 27 de fixation par serrage de la poignée 24 ou analogue et, d'autre part, une sous-structure 29" venant en engagement avec les ensembles 32, 32' de guidage et d'entraînement, par exemple sous la forme d'un jeu de rails 33, 33' incurvés de la sous-structure 29" circulant entre des arrangements 32, 32' de rouleaux ou de galets de guidage ou d'entraînement.

Préférentiellement, et comme le montre la figure 13, les moyens de déplacement en rotation du châssis porteur 29 peuvent comprendre deux ensembles 32 de galets de guidage coopérant chacun avec un rail 33 correspondant et un ensemble 32' de galets ou de pignons d'entraînement venant en engagement avec un rail denté 33'.

Les guidages et l'entraînement circulaires fournis par les ensembles 32, 32' associés aux rails 33, 33' permettent de déporter ces fonctions, évitant ainsi d'encombrer l'espace autour de l'axe de rotation/translation AL' et permettant d'y installer le système endoscopique.

Dans le cas de la variante selon les figures 11A et 11B et de manière similaire à la réalisation de la figure 12, il peut en outre être prévu que la platine 25 soit constituée de deux parties mobiles en translation l'une par rapport à l'autre, ledit déplacement étant éventuellement contrôlé et motorisé (repères 25, 26 sur les figures 11).

En accord avec une caractéristique supplémentaire de l'invention, non spécifiquement représentée, il peut être prévu que le corps allongé flexible 23 comporte également au moins un canal de vision.

En outre, à chaque moyen d'actionnement ou d'entraînement 7, 7', 15, 18, 26 de la ou chaque unité opérationnelle 2 et de l'unité ou poignée de commande 24 peut être associé un capteur de déplacement, les signaux visuels et les signaux des capteurs étant transmis à une unité informatique d'évaluation et de pilotage, pourvue de moyens d'interfaçage homme-machine.

Cette unité informatique peut correspondre ou comprendre le système de pilotage commun à l'ensemble des composantes du système endoscopique.

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Dispositif **(1)** d'instrumentation motorisé et modulable pour un endoscope ou analogue, en particulier un endoscope flexible, comprenant au moins une unité opérationnelle **(2)** montée sur au moins une structure support **(3),** préférentiellement articulée, la ou chaque unité **(2)** intégrant un module d'instrument médical motorisé **(4)** comprenant, d'une part, un instrument médical **(5)** allongé avec un effecteur ou outil **(6)** disposé à une extrémité distale, la portion d'extrémité **(5')** correspondante de l'instrument **(5)** étant apte à subir une flexion ou un cintrage dans au moins un plan et/ou selon au moins une direction et, d'autre part, au moins un moyen d'actionnement motorisé **(7, 7')** commandant le fonctionnement de l'outil ou de l'effecteur et/ou la flexion ou le cintrage de la portion d'extrémité distale **(5'),** ce par l'intermédiaire de moyens de transmission **(8, 8')** s'étendant dans le corps allongé **(9)** de l'instrument médical, ce dernier étant en outre apte à être soumis à des déplacements en translation dans la direction de son axe longitudinal **(AL)** et à des déplacements en rotation autour de cet axe, le ou les moyen(s) d'actionnement **motorisé(s)** (7, 7') étant monté(s) dans un
corps creux (10) relié à l'extrémité proximale (5") de l'instrument médical (5), **caractérisé en ce que** ledit corps creux (10) est lui-même disposé dans un boîtier de réception (11) avec guidage en rotation autour d'un axe médian
propre (AM) prolongeant l'axe longitudinal (AL) de l'instrument médical (5) **du côté de son extrémité proximale (5"), et en ce que ledit boîtier de réception (11) est** monté avec faculté de déplacement, au moins en translation selon la direction de l'axe médian (AM) du corps creux (10), sur la structure support (3),
les déplacements du corps creux (10) et du boîtier de réception (11) étant motorisés.

2. Dispositif selon la revendication 1, **caractérisé en ce que** le corps creux (10), qui délimite avantageusement une enceinte sensiblement étanche, présente, au moins extérieurement, une structure de révolution autour de son axe médian (AM), avantageusement cylindrique circulaire et préférentiellement en forme d'obus avec une extrémité effilée (10') constituant une interface de raccordement avec le corps (9) de l'instrument médical (5), et **en ce que** le boîtier de réception (11) comporte un arrangement d'au moins deux paliers à roulement (12) espacés, à structure continue ou discontinue, assurant le guidage en rotation du corps creux (10) dans le boîtier (11).

3. Dispositif selon la revendication 1 ou 2, **caractérisé en ce que** le boîtier de réception (11) consiste en une coque en au moins deux parties (11', 11 "), par exemple deux parties mutuellement articulées dont l'une (11") forme un couvercle, autorisant l'introduction et l'extraction d'un corps creux (10) en position ouverte, ladite coque comportant deux ouvertures opposées (13, 13') destinées respectivement, d'une part, au passage de l'instrument médical (5) ou d'une partie d'extrémité (10') du corps creux (10), et, d'autre part, au passage des lignes (14) d'alimentation et/ou de commande du ou des moyen(s) d'actionnement (7, 7') commandant l'outil (6) et/ou l'extrémité distale (5') flexible.

4. Dispositif selon l'une quelconque des revendications 1 à 3, **caractérisé en ce que** le boîtier de réception (11) comporte un mécanisme motorisé (15) d'entraînement contrôlé en rotation du corps creux (10) autour de son axe médian (AM), par exemple formé d'un pignon denté motorisé (16) engrenant avec une couronne dentée (16') s'étendant périphériquement autour dudit corps creux (10), la liaison entre ce dernier et l'instrument médical (5) étant apte à transmettre cette rotation au corps allongé (9) dudit instrument (5).

5. Dispositif selon l'une quelconque des revendications 1 à 4, **caractérisé en ce que** le ou chaque boîtier de réception (11) est monté sur la ou une structure support (3) par l'intermédiaire d'une liaison coulissante (17), par exemple une liaison à glissière, à coulisseau ou à chariot, sensiblement selon la direction de l'axe longitudinal (AL) de l'instrument médical allongé (5) qu'il renferme, le déplacement du boîtier de réception (11) par rapport à la structure support (3) concernée étant contrôlé par un mécanisme d'entraînement motorisé (18), préférentiellement intégré audit boîtier (11).

6. Dispositif selon l'une quelconque des revendications 1 à 5, **caractérisé en ce que** la ou chaque structure support (3) consiste en un bras articulé dont une extrémité est pourvue d'un organe (20) de fixation ou d'assemblage rigide et amovible avec un équipement d'une salle d'opération, par exemple une table d'opération, et dont l'autre extrémité porte au moins un, préférentiellement deux, boîtier(s) de réception (11), par exemple par l'intermédiaire d'une entretoise support ou d'une pièce analogue (21) autorisant un montage orienté et éventuellement mutuellement espacé, lorsque plusieurs boîtiers de réception (11) sont présents.

7. Dispositif selon l'une quelconque des revendications 1 à 6, **caractérisé en ce que** le corps creux (10) intègre des moyens d'actionnement (7') et des moyens de transmission (8') permettant de réaliser une flexion ou un cintrage de l'extrémité distale (5') de l'instrument médical (5) dans deux plans sécants, préférentiellement perpendiculaires entre eux.

8. Dispositif selon la revendication 3 ou la revendication 5, lorsqu'elle dépend de la revendication 3, **caractérisé en ce qu'**il comprend également, pour chaque module d'instrument (4) et éventuellement pour chaque boîtier de réception (11), une unité de commande (19) dédiée pour le contrôle des moyens d'actionnement (7, 7') et, le cas échéant, du mécanisme d'entraînement (18) et/ou du mécanisme (15) de rotation motorisée, la ou chaque unité de commande étant éventuellement asservie et/ou apte à traiter des signaux de mesure fournis par un ou des capteur(s) ou détecteur(s).

9. Système endoscopique flexible comprenant, d'une part, un corps allongé (23) avec au moins un, préférentiellement au moins deux, canal(aux) fonctionnel(s) longitudinal(aux) (22') destiné(s) à recevoir chacun un instrument médical (5) dont l'extrémité distale portant l'effecteur ou l'outil (6) peut déboucher de l'extrémité distale dudit corps allongé (23) et, d'autre part, une unité ou poignée de commande (24) reliée à l'extrémité proximale du corps allongé et comportant, pour le ou chaque instrument médical, une ouverture d'introduction (24') se prolongeant par un canal longitudinal correspondant, ladite unité ou poignée (24) intégrant des moyens d'actionnement contrôlant la flexion ou le cintrage de la portion d'extrémité distale (23') au moins du corps allongé, système endoscopique (22) **caractérisé en ce qu'**il comprend en outre au moins un dispositif (1) d'instrumentation motorisé et modulable selon l'une quelconque des revendications 1 à 8, dont l'instrument médical (5) est disposé dans le ou un canal fonctionnel (22') avec faculté de déplacement en translation et en rotation.

10. Système endoscopique selon la revendication 9, **caractérisé en ce que** l'unité ou poignée de commande (24) est fixée sur une platine de montage (25), dont le positionnement spatial est réglable grâce à une structure support (28, 3) articulée, ladite poignée (24) pouvant être déplacée en translation, sensiblement selon la direction de l'axe longitudinal (AL') du corps allongé flexible (23), de manière contrôlée, préférentiellement motorisée.

11. Système endoscopique selon la revendication 10, **caractérisé en ce que** la platine de montage (25) comprend une embase (25') solidarisée à la structure support (28, 3) et un chariot (25") comportant un ou des moyen(s) (27) de fixation rigide et amovible pour la poignée (24), le chariot (25") étant guidé en translation par rapport à l'embase (25') et déplacé par rapport à ce dernier grâce à un moyen (26) d'entraînement motorisé, par exemple sous la forme d'un pignon motorisé (26') engrenant soit avec un rail denté ou une crémaillère solidaire du ou formé sur le chariot (25"), soit avec une courroie crantée (26") déplaçant ledit chariot (25").

12. Système endoscopique selon l'une quelconque des revendications 9 et 10, **caractérisé en ce que** les déplacements du ou des module(s) d'instrument médical (4) et ceux de la poignée de commande (24) sont mutuellement asservis.

13. Système endoscopique selon l'une quelconque des revendications 10 et 11, **caractérisé en ce que** la platine (25) est portée par une structure support (28) distincte et indépendante de la ou des structure(s) support(s) (3) portant le ou les dispositif(s) d'instrumentation (1), par exemple par un bras articulé dont une extrémité est pourvue d'un organe (28') pour la fixation ou l'assemblage rigide et amovible avec un équipement d'une salle d'opération, par exemple une table d'opération.

14. Système endoscopique selon l'une quelconque des revendications 10 et 11, **caractérisé en ce que** la platine (25) ou ledit au moins un moyen de fixation (27) est porté(e) par la même structure support (3) que celle portant le ou les boîtiers de réception (11) du dispositif d'instrumentation (1) selon l'une quelconque des revendications 1 à 8.

15. Système endoscopique selon l'une quelconque des revendications 9 à 14, **caractérisé en ce que** le ou chaque dispositif d'instrumentation (1) comprend au moins deux unités opérationnelles (2), dont les boîtiers de réception (11) sont montés sur une entretoise (21) ou analogue par l'intermédiaire d'une liaison coulissante (17), préférentiellement motorisée, la disposition relative des unités opérationnelles (2) par rapport à la poignée (24) ou analogue, maintenue par un moyen de fixation (27), éventuellement solidaire d'une platine (25) avec au moins un degré de liberté, étant réglée de telle manière que les axes médians (AM) des corps creux (10) sont sensiblement alignés avec les ouvertures (24') d'introduction des instruments médicaux (5) de la poignée (24) ou analogue.

16. Système endoscopique selon l'une quelconque des revendications 9 à 15, **caractérisé en ce que** ledit au moins un dispositif (1) d'instrumentation motorisé et modulable et ladite unité ou poignée de commande (24) sont portés par une unique structure support (3), par exemple sous la forme d'un bras ou d'un pied articulé, préférentiellement à articulations multiples, et **en ce que** ledit dispositif (1) et ladite poignée (24) sont montés sur ladite structure support (3) par l'intermédiaire d'un châssis porteur (29) fournissant une configuration de positionnement mutuel adaptée pour les unités opérationnelles (2) et la poignée (24) et pouvant être déplacé, de manière contrôlée, en rotation et en translation par rapport à la structure support (3), préférentiellement sensiblement autour et selon l'axe longitudinal (AL') du corps allongé (23) du système endoscopique (22).

17. Système endoscopique selon la revendication 16, **caractérisé en ce que** les moyens de déplacement du châssis porteur (29) par rapport à la structure support (3) comprennent, d'une part, un chariot ou un patin (30) mobile en translation, de manière motorisée, par rapport à une platine (31) solidaire de la structure support (3), et, d'autre part, des ensembles (32, 32') de guidage et d'entraînement en rotation du châssis porteur (29) fixés sur ce chariot ou patin (30).

18. Système endoscopique selon la revendication 17, **caractérisé en ce que** le châssis support (29) comprend, d'une part, une superstructure (29') sur laquelle sont installés, éventuellement par l'intermédiaire d'une liaison articulée pouvant être bloquée en position, une entretoise support (21) ou analogue pour les unités opérationnelles (2) et le moyen (27) de fixation par serrage de la poignée (24) ou analogue et, d'autre part, une sous-structure (29") venant en engagement avec les ensembles (32, 32') de guidage et d'entraînement, par exemple sous la forme d'un jeu de rails (33, 33') incurvés de la sous-structure (29") circulant entre des arrangements (32, 32') de rouleaux ou de galets de guidage ou d'entraînement.

19. Système endoscopique selon l'une quelconque des revendications 9 à 18, **caractérisé en ce que** le corps allongé flexible (23) comporte également au moins un canal de vision et **en ce qu'**à chaque moyen d'actionnement ou d'entraînement (7, 7', 15, 18, 26) de la ou chaque unité opérationnelle (2) et de l'unité ou poignée de commande (24) est associé un capteur de déplacement, les signaux visuels et les signaux des capteurs étant transmis à une unité informatique d'évaluation et de pilotage, pourvue de moyens d'interfaçage homme-machine.

## Patentansprüche

1. Motorisierte und modulare Instrumentierungsvorrichtung (1) für ein Endoskop oder dergleichen, insbesondere für ein flexibles Endoskop, mindestens eine Arbeitseinheit (2) umfassend, die auf mindestens einer vorzugsweise gelenkigen Tragekonstruktion (3) angebracht ist, wobei die oder jede Einheit (2) ein motorisiertes medizinisches Instrumentenmodul (4) beinhaltet, das einerseits ein langgestrecktes medizinisches Instrument (5) umfasst mit einem Wirkelement oder Werkzeug (6), das an einem distalen Ende angebracht ist, wobei der entsprechende Endabschnitt (5') des Instrumentes (5) in der Lage ist, eine Krümmung oder Biegung in mindestens einer Ebene und/oder in mindestens einer Richtung zu erfahren, und andererseits mindestens ein motorisiertes Betätigungsmittel (7, 7'), das die Arbeit des Werkzeugs oder Wirkelements und/oder die Krümmung oder Biegung des distalen Endabschnitts (5') mit Hilfe von Übertragungsmitteln (8, 8') bewirkt, die im langgestreckten Körper (9) des medizinischen Instrumentes verlaufen, wobei das letztgenannte außerdem in der Lage ist, Translationsbewegungen in Richtung seiner Längsachse (AL) und Drehbewegungen um diese Achse unterworfen zu werden,
wobei das oder die motorisierte(n) Betätigungsmittel (7, 7') in einem Hohlkörper (10) montiert ist/sind, der mit dem proximalen Ende (5") des medizinischen Instrumentes (5) verbunden ist, **dadurch gekennzeichnet, dass** der genannte Hohlkörper (10) seinerseits in einem Aufnahmegehäuse (11) mit Führung zur Drehung um eine eigene Mittelachse (AM), die die Längsachse (AL) des medizinischen Instrumentes (5) auf der Seite seines proximalen Endes (5") fortsetzt, angeordnet ist und
dadurch, dass das genannte Aufnahmegehäuse (11) mit der Möglichkeit zur Bewegung mindestens in Translation in der Richtung der Mittelachse (AM) des Hohlkörpers (10) auf der Tragekonstruktion (3) angebracht ist,
wobei die Bewegungen des Hohlkörpers (10) und des Aufnahmegehäuses (11) motorisiert sind.

2. Vorrichtung nach Patentanspruch 1, **dadurch gekennzeichnet, dass** der Hohlkörper (10), der vorteilhafterweise einen im Wesentlichen dichten Raum umgibt, mindestens außen eine rotationssymmetrische Form um seine Mittelachse (AM) aufweist, vorteilhafterweise kreisförmig zylindrisch und vorzugsweise in Spitzbogenform mit einem konisch zulaufenden Ende (10'), das ein Anschlussstück zur Befestigung am Körper (9) des medizinischen Instrumentes (5) bildet, und dadurch, dass das Aufnahmegehäuse (11) eine Anordnung von mindestens zwei Wälzlagern (12), mit einteiligem oder geteiltem Aufbau mit Abstand voneinander aufweist, die die Führung der Drehung des Hohlkörpers (10) im Gehäuse (11) sicherstellen.

3. Vorrichtung nach Patentanspruch 1 oder 2, **dadurch gekennzeichnet, dass** das Aufnahmegehäuse (11) aus einer Schale aus mindestens zwei Teilen (11', 11 ") besteht, beispielsweise zwei aneinander angelenkten Teilen, von denen einer (11") einen Deckel bildet, der in der offenen Stellung das Einsetzen und die Entnahme eines Hohlkörpers (10) erlaubt, wobei die genannte Schale zwei gegenüberliegende Öffnungen (13, 13') aufweist, die einerseits für den Durchtritt des medizinischen Instrumentes (5) oder eines Endteils (10') des Hohlkörpers (10) dienen, bzw. andererseits für den Durchtritt der Versorgungs- (14) und/oder Steuerleitungen des oder der Betätigungsmittel (7, 7'), die das Werkzeug (6) und/oder das flexible distale Ende (5') steuern.

4. Vorrichtung nach irgendeinem der Patentansprüche 1 bis 3, **dadurch gekennzeichnet, dass** das Aufnahmegehäuse (11) einen motorisierten Mechanismus (15) zum gesteuerten Antrieb des Hohlkörpers (10) zu Drehungen um seine Mittelachse (AM) enthält, beispielsweise in Form eines motorisierten Zahnrades (16), das mit einem Zahnkranz (16') in Eingriff steht, der peripher um den genannten Hohlkörper (10) verläuft, wobei die Verbindung zwischen dem letztgenannten und dem medizinischen Instrument (5) geeignet ist, diese Drehung auf den langgestreckten Körper (9) des genannten Instrumentes (5) zu übertragen.

5. Vorrichtung nach irgendeinem der Patentansprüche 1 bis 4, **dadurch gekennzeichnet, dass** das oder jedes Aufnahmegehäuse (11) über eine im Wesentlichen in der Richtung der Längsachse (AL) des langgestreckten, medizinischen Instrumentes, das es enthält, verschiebbare Verbindung (17), beispielsweise eine Gleitschienen-, Schieber- oder Wagenverbindung, auf der oder einer Tragekonstruktion (3) angebracht ist, wobei die Verschiebung des Aufnahmegehäuses (11) relativ zur betreffenden Tragekonstruktion (3) durch einen motorisierten Antriebsmechanismus (18) gesteuert wird, der vorzugsweise in das genannte Gehäuse (11) eingebaut ist.

6. Vorrichtung nach irgendeinem der Patentansprüche 1 bis 5, **dadurch gekennzeichnet, dass** die oder jede Tragekonstruktion (3) aus einem gelenkigen Arm besteht, dessen eines Ende mit einem Organ (20) zur starren und abnehmbaren Befestigung an oder zum Zusammensetzen mit der Ausstattung eines Operationssaales, beispielsweise eines Operationstisches, versehen ist und dessen anderes Ende mindestens ein, vorzugsweise zwei, Aufnahmegehäuse (11) trägt, beispielsweise über eine Tragestrebe oder ein ähnliches Teil (21), das eine gerichtete und eventuell voneinander beabstandete Montage erlaubt, wenn mehrere Aufnahmegehäuse (11) vorhanden sind.

7. Vorrichtung nach irgendeinem der Patentansprüche 1 bis 6, **dadurch gekennzeichnet, dass** der Hohlkörper (10) Betätigungsmittel (7') und Übertragungsmittel (8') enthält, die eine Krümmung oder Biegung des distalen Endes (5') des medizinischen Instrumentes (5) in zwei einander schneidenden Ebenen, die vorzugsweise senkrecht zueinander stehen, erlauben.

8. Vorrichtung nach Patentanspruch 3 oder Patentanspruch 5, wenn er von Patentanspruch 3 abhängig ist, **dadurch gekennzeichnet, dass** sie ebenfalls für jedes Instrumentenmodul (4) und eventuell für jedes Aufnahmegehäuse (11) eine Steuereinheit (19) umfasst, die der Steuerung der Betätigungsmittel (7, 7') und gegebenenfalls des Antriebsmechanismus (18) und/oder des motorisierten Drehmechanismus (15) dient, wobei die oder jede Steuereinheit eventuell geführt wird und/oder in der Lage ist, Messsignale zu verarbeiten, die von einem oder mehreren Sensor(en) oder Detektor(en) abgegeben werden.

9. Flexibles Endoskopsystem, umfassend: einerseits einen langgestreckten Körper (23) mit mindestens einem, vorzugsweise mindestens zwei Funktionskanal/kanälen (22') in Längsrichtung, dazu bestimmt, jeweils ein medizinisches Instrument (5) aufzunehmen, dessen distales Ende, das das Wirkelement oder Werkzeug (6) trägt, aus dem distalen Ende des genannten langgestreckten Körpers (23) ausmünden kann, und andererseits eine Steuereinheit oder -griff (24), die mit dem proximalen Ende des langgestreckten Körpers verbunden ist und für das oder jedes medizinische Instrument eine Einführöffnung (24') aufweist, die sich in einem entsprechenden Kanal in Längsrichtung fortsetzt, wobei die genannte Einheit oder Griff (24) Betätigungsmittel aufweist, die die Krümmung oder Biegung mindestens des distalen Endabschnittes (23') des langgestreckten Körpers steuert, Endoskopsystem (22), **dadurch gekennzeichnet, dass** es außerdem mindestens eine motorisierte und modulare Instrumentierungsvorrichtung (1) nach irgendeinem der Patentansprüche 1 bis 8 umfasst, dessen medizinisches Instrument (5) in dem oder einem Funktionskanal (22') mit der Möglichkeit zu Verschiebungs- und Drehbewegungen angeordnet ist.

10. Endoskopsystem nach Patentanspruch 9, **dadurch gekennzeichnet, dass** die Steuereinheit oder -griff (24) auf einer Montageplatte (25) befestigt ist, deren räumliche Stellung mit Hilfe einer gelenkigen Tragekonstruktion (28, 3) einstellbar ist, wobei der genannte Griff (24) im Wesentlichen in der Richtung der Längsachse (AL') des flexiblen, langgestreckten Körpers (23) gesteuert und vorzugsweise motorisiert versetzt werden kann.

11. Endoskopsystem nach Patentanspruch 10, **dadurch gekennzeichnet, dass** die Montageplatte (25) einen Sockel (25') umfasst, der an der Tragekonstruktion (28, 3) und einem Wagen (25") befestigt ist, der ein oder mehrere Mittel (27) zur starren und abnehmbaren Befestigung des Griffes (24) aufweist, wobei der Wagen (25") für Translationsbewegungen relativ zum Sockel (25') geführt ist und relativ zum letztgenannten durch ein motorisiertes Antriebsmittel (26), beispielsweise in Form eines motorisierten Zahnrades (26'), bewegt wird, das entweder mit einer Zahnschiene oder einer Zahnstange, die mit dem Wagen (25") fest verbunden ist oder ihm angeformt ist, oder einem Zahnriemen (26") in Eingriff steht, der den genannten Wagen (25") bewegt.

12. Endoskopsystem nach irgendeinem der Patentansprüche 9 und 10, **dadurch gekennzeichnet, dass** die Bewegungen des oder der medizinischen Instrumentenmoduls/e (4) und die des Steuergriffes (24) voneinander abhängig sind.

13. Endoskopsystem nach irgendeinem der Patentansprüche 10 und 11, **dadurch gekennzeichnet, dass** die Platte (25) von einer Tragekonstruktion (28) getragen wird, die von der oder den Tragekonstruktion(en) (3), die die Instrumentierungsvorrichtungen (1) trägt oder tragen, verschieden und unabhängig ist, beispielsweise von einem gelenkigen Arm, dessen eines Ende mit einem Organ (28') zur starren und abnehmbaren Befestigung an oder zum Zusammensetzen mit einer Ausstattung eines Operationssaales, beispielsweise einem Operationstisch, versehen ist.

14. Endoskopsystem nach irgendeinem der Patentansprüche 10 und 11, **dadurch gekennzeichnet, dass** die Platte (25) oder das genannte mindestens eine Befestigungsmittel (27) von derselben Tragekonstruktion (3) getragen wird, wie der, die das oder die Aufnahmegehäuse (11) der Instrumentierungsvorrichtung (1) nach irgendeinem der Patentansprüche 1 bis 8 trägt.

15. Endoskopsystem nach irgendeinem der Patentansprüche 9 bis 14, **dadurch gekennzeichnet, dass** die oder jede Instrumentierungsvorrichtung (1) mindestens zwei Arbeitseinheiten (2) umfasst, deren Aufnahmegehäuse (11) auf einer Strebe (21) oder dergleichen über eine vorzugsweise motorisierte verschiebbare Verbindung (17) montiert sind, wobei die relative Anordnung der Arbeitseinheiten (2) gegenüber dem Griff (24) oder Ähnlichem durch ein Befestigungsmittel (27) aufrechterhalten wird, das gegebenenfalls an einer Platte (25) mit mindestens einem Freiheitsgrad befestigt ist und derart eingestellt ist, dass die Mittelachsen (AM) der Hohlkörper (10) mit den Einführöffnungen (24') der medizinischen Instrumente (5) des Griffes (24) oder dergleichen im Wesentlichen ausgerichtet sind.

16. Endoskopsystem nach irgendeinem der Patentansprüche 9 bis 15, **dadurch gekennzeichnet, dass** die genannte mindestens eine motorisierte und modulare Instrumentierungsvorrichtung (1) und die genannte Steuereinheit oder -griff (24) von einer einzigen Tragekonstruktion (3) getragen werden, beispielsweise in Form eines gelenkigen Armes oder Ständers, vorzugsweise mit mehrfachen Gelenken, und dadurch, dass die genannte Vorrichtung (1) und der genannte Griff (24) über einen Tragrahmen (29) auf der genannten Tragekonstruktion (3) montiert sind, der zu einer Konfiguration der Positionierung zueinander führt, die den Arbeitseinheiten (2) und dem Griff (24) angepasst sind und der gesteuert in Drehung und Translationsbewegung relativ zur Tragekonstruktion (3) bewegt werden kann, vorzugsweise im Wesentlichen um und längs der Längsachse (AL') des langgestreckten Körpers (23) des Endoskopsystems (22).

17. Endoskopsystem nach Patentanspruch 16, **dadurch gekennzeichnet, dass** die Mittel zur Bewegung des Tragrahmens (29) relativ zur Tragekonstruktion (3) einerseits einen gegenüber einer an der Tragekonstruktion (3) befestigten Platte (31) motorisiert translationsbeweglichen Wagen oder Schlitten (30) umfasst und andererseits auf dem Wagen oder Schlitten (30) befestigte Führungs- und Antriebsanordnungen (32. 32') zur Drehung des Tragrahmens (29).

18. Endoskopsystem nach Patentanspruch 17, **dadurch gekennzeichnet, dass** der Tragrahmen (29) einerseits einen Überbau (29') umfasst, auf dem eventuell über eine Gelenkverbindung, die in ihrer Stellung verriegelt werden kann, eine Tragstrebe (21) oder dergleichen für die Arbeitseinheiten (2) und das Mittel (27) zur Befestigung durch Festspannen des Griffes (24) oder dergleichen montiert ist, und andererseits einen Unterbau (29"), der mit den Führungs- und Antriebsanordnungen (32, 32') in Eingriff gelangt, beispielsweise in Form eines gekrümmten Schienensatzes (33, 33') des Unterbaus (29"), die zwischen Führungs- oder Antriebsrollenanordnungen (32, 32') laufen.

19. Endoskopsystem nach irgendeinem der Patentansprüche 9 bis 18, **dadurch gekennzeichnet, dass** der langgestreckte, flexible Körper (23) auch mindestens einen Sichtkanal aufweist und dadurch, dass jedem Betätigungs- oder Antriebsmittel (7, 7', 15, 18, 26) der oder jeder Arbeitseinheit (2) und der Steuereinheit oder des Steuergriffes (24) ein Bewegungssensor zugeordnet ist, wobei die visuellen Signale und die Sensorsignale einer Datenverarbeitungseinheit zur Auswertung und Lenkung übermittelt werden, die mit Mensch-Maschine-Schnittstellenmitteln versehen ist.

## Claims

1. Motorised and modular instrumentation device (1) for an endoscope or the like, in particular a flexible endoscope, comprising at least one operational unit (2) mounted on at least one support structure (3), preferably an articulated support structure, with the unit or each unit (2) integrating a motorised medical instrument module (4) comprising, on the one hand, an elongated medical instrument (5) with an actuator or a tool (6) placed at a distal end, with the corresponding end portion (5') of the instrument (5) being able to undergo bending or arching in at least one plane and/or in at least one direction and, on the other hand, at least one motorised actuating means (7, 7') controlling the operating of the tool or the actuator and/or the bending or the arching of the distal end portion (5'), where this is done by transmission means (8, 8') extending into the elongated body (9) of the medical instrument, the latter also being able to be subjected to translational movements in the direction of its longitudinal axis (AL) and to rotational movements around this axis,
the actuating means (7, 7') being mounted in a hollow body (10) connected to the proximal end (5") of the medical instrument (5), **characterised in that** said hollow body (10) is itself placed with guiding in rotation around a suitable median axis (AM) extending the longitudinal axis (AL) of the medical instrument (5) in a receiving housing (11), and **in that** said receiving housing (11) is mounted with the ability to move at least in translation in the direction of the median axis (AM) of the hollow body (10), on the support structure (3), with the movements of the hollow body (10) and of the receiving housing (11) being motorised.

2. Device according to claim 1, **characterised in that** the hollow body (10), which advantageously delimits an essentially airtight chamber, has, at least on the outside, a structure for rotation around its median axis (AM), advantageously circular cylindrical and preferably in the form of a shell with a tapered end (10') constituting a connecting interface with the body (9) of the medical instrument (5), and wherein the receiving housing (11) comprises an arrangement of at least two spaced roller bearings (12) with a continuous or discontinuous structure, ensuring the guiding in rotation of the hollow body (10) in the housing (11).

3. Device according to claim 1 or 2, **characterised in that** the receiving housing (11) consists of a shell made in at least two parts (11', 11 "), for example two mutually articulated parts, one (11") of which forms a cover, allowing the introduction and the extraction of a hollow body (10) in an open position, with said shell comprising two opposing openings (13, 13') respectively intended, on the one hand, for the passage of the medical instrument (5) or a portion of the end (10') of the hollow body (10), and, on the other hand, for the passage of lines (14) for supply and/or control of the actuating means (7, 7') controlling the tool (6) and/or the flexible distal end (5').

4. Device according to any one of claims 1 to 3, **characterised in that** the receiving housing (11) comprises a motorised mechanism (15) for controlled driving in rotation of the hollow body (10) around its median axis (AM), for example formed by a motorised cogwheel (16) engaging with a ring gear (16') extending peripherally around said hollow body (10), with the connection between the latter and the medical instrument (5) being able to transmit this rotation to the elongated body (9) of said instrument (5).

5. Device according to any one of claims 1 to 4, **characterised in that** the receiving housing (11) or each receiving housing (11) is mounted on the support structure (3) or a support structure (3) by means of a sliding connection (17), for example a slide connection, a carriage connection or a trolley connection, essentially in the direction of the longitudinal axis (AL) of the elongated medical instrument (5) that it contains, with the movement of the receiving housing (11) relative to the support structure (3) in question being controlled by a motorised drive mechanism (18), preferably integrated into said housing (11).

6. Device according to any one of claims 1 to 5, **characterised in that** the support structure (3) or each support structure (3) consists of an articulated arm, one end of which is equipped with an element (20) for attachment or rigid and removable assembly with a piece of equipment from an operating room, for example an operating table, and the other end of which carries at least one, preferably two, receiving housing(s) (11), for example by means of a support crosspiece or an analogous part (21) allowing an oriented and optionally mutually spaced mounting when several receiving housings (11) are present.

7. Device according to any one of claims 1 to 6, **characterised in that** the hollow body (10) integrates actuating means (7') and transmitting means (8') making it possible to carry out bending or arching of the distal end (5') of the medical instrument (5) in two secant planes, preferably perpendicular to one another.

8. Device according to claim 3 or claim 5, when it is dependent on claim 3, **characterised in that** it also comprises, for each instrument module (4) and optionally for each receiving box (11), a dedicated control unit (19) for the control of the actuating means (7, 7') and, if necessary, the drive mechanism (18) and/or the motorised rotation mechanism (15), with the control unit or each control unit preferably being slaved and/or able to process measurement signals provided by one or more sensor(s) or detector(s).

9. Flexible endoscopic system comprising, on the one hand, an elongated body (23) with at least one, preferably at least two, longitudinal functional channel(s) (22'), each intended to accommodate a medical instrument (5) the distal end of which that supports the actuator or the tool (6) can emerge from the distal end of said elongated body (23), and, on the other hand, a control unit or control grip (24) connected to the proximal end of the elongated body and comprising, for the medical instrument or for each medical instrument, an introduction opening (24') that extends through a corresponding longitudinal channel, with said unit or grip (24) integrating actuating means controlling the bending or the arching of the distal end portion (23') at least of the elongated body, endoscopic system (22) **characterised in that** it comprises in addition at least one motorised and modular instrumentation device (1) according to any one of claims 1 to 8, the medical instrument (5) of which is placed in the functional channel (22') or a functional channel (22') with the ability to move in translation and in rotation.

10. Endoscopic system according to claim 9, **characterised in that** the control unit or control grip (24) is attached to a mounting plate (25), whose spatial positioning can be adjusted using an articulated support structure (28, 3), whereby said grip (24) can be moved in translation, essentially in the direction of the longitudinal axis (AL') of the flexible elongated body (23), in a controlled, preferably motorised, manner.

11. Endoscopic system according to claim 10, **characterised in that** the mounting plate (25) comprises a base (25') that is made integral with the support structure (28, 3) and a trolley (25") comprising rigid and removable attachment means (27) for the grip (24), with the trolley (25") being guided in translation relative to the base (25') and moved relative to the latter owing to a motorised drive means (26), for example in the form of a motorised pinion (26') engaging either with a toothed rail or a rack that is integral with or formed on the trolley (25") or with a notched belt (26") moving said trolley (25").

12. Endoscopic system according to any one of claims 9 and 10, **characterised in that** the movements of the medical instrument module(s) (4) and those of the control grip (24) are mutually slaved.

13. Endoscopic system according to any one of claims 10 and 11, **characterised in that** the plate (25) is carried by a support structure (28) that is separate and independent from the support structure(s) (3) carrying the instrumentation device(s) (1), for example by an articulated arm whose one end is equipped with an element (28') for the attachment or the rigid and removable assembly with a piece of equipment from an operating room, for example an operating table.

14. Endoscopic system according to any one of claims 10 and 11, **characterised in that** the plate (25) or said at least one attachment means (27) is carried by the same support structure (3) as the one carrying the receiving housing(s) (11) of the instrumentation device (1) according to any one of claims 1 to 8.

15. Endoscopic system according to any one of claims 9 to 14, **characterised in that** the instrumentation device (1) or each instrumentation device (1) comprises at least two operational units (2), the receiving housings (11) of which are mounted on a crosspiece (21) or the like by means of a sliding connection (17), preferably motorised, with the relative arrangement of the operational units (2), relative to the grip (24) or the like, held by an attachment means (27) that is optionally integral with a plate (25) with at least one degree of freedom, being regulated in such a way that the median axes (AM) of the hollow bodies (10) are essentially aligned with the openings (24') for introduction of the medical instruments (5) of the grip (24) or the like.

16. Endoscopic system according to any one of claims 9 to 15, **characterised in that** said at least one motorised and modular instrumentation device (1) and said control unit or control grip (24) are carried by a single support structure (3), for example in the form of an articulated arm or foot, preferably with multiple articulations, and wherein said device (1) and said grip (24) are mounted on said support structure (3) by means of a support chassis (29) providing a suitable mutual positioning configuration for the operational units (2) and the grip (24) and can be moved, in a controlled manner, in rotation and in translation relative to the support structure (3), preferably essentially around and along the longitudinal axis (AL') of the elongated body (23) of the endoscopic system (22).

17. Endoscopic system according to claim 16, **characterised in that** the means for movement of the support chassis (29) relative to the support structure (3) comprise, on the one hand, a trolley or a pad (30) that can move in translation, in a motorised manner, relative to a plate (31) that is integral with the support structure (3), and, on the other hand, assemblies (32, 32') for guiding and driving in rotation the support chassis (29) attached to this trolley or pad (30).

18. Endoscopic system according to claim 17, **characterised in that** the support chassis (29) comprises, on the one hand, a superstructure (29') on which are installed, optionally by means of an articulated connection that can be locked in position, a support crosspiece (21) or the like for the operational units (2) and the means (27) for attachment by tightening of the grip (24) or the like, and, on the other hand, a substructure (29") that engages with the assemblies (32, 32') for guiding and driving, for example in the form of a set of rails (33, 33') curved from the substructure (29") running between arrangements (32, 32') of rollers or guiding or drive tensioners.

19. Endoscopic system according to any one of claims 9 to 18, **characterised in that** the flexible elongated body (23) also comprises at least one viewing channel and **in that** a movement sensor is combined with each means (7, 7', 15, 18, 26) for actuating or driving the operational unit (2) or each operational unit (2) and the control unit or control grip (24), with the visual signals and the signals from sensors being transmitted to a computer unit for evaluation and control, equipped with man-machine interfaces.
